# FASCICULE DE BREVET EUROPEEN

(11) **EP 0 679 375 B1**
(45) Date de publication et mention de la délivrance du brevet: **02.09.1998**
(21) Numéro de dépôt: 95420090.3
(22) Date de dépôt: 10.04.1995
(51) Int. Cl.: A61F 2/40

(54) **Ensemble prothétique modulaire pour l'articulation de l'épaule**
Modularer Prothesensatz für das Schultergelenk
Modular prosthetic set for the shoulder joint

(30) Priorité: 25.04.1994 FR 9405329
(43) Date de publication de la demande: 02.11.1995
(73) Titulaire: EUROS Société Anonyme, F-13600 La Ciotat (FR)
(72) Inventeur: Odella, Ferdinando, Milan (IT)
(74) Mandataire: Somnier, Jean-Louis

(56) Documents cités:
- EP-A- 0 038 897
- EP-A- 0 339 530
- EP-A- 0 428 303
- EP-A- 0 549 480
- EP-A- 0 592 897
- WO-A-93/09733
- WO-A-94/15551
- FR-A- 2 664 809
- FR-A- 2 699 400

## Description

L'invention se rattache au secteur technique des prothèses d'épaules.

D'une manière parfaitement connue pour un homme du métier, ce type de prothèses comprend une tige humérale apte à être ancrée dans l'humérus, avec ou non un ciment orthopédique. Cette tige présente, au niveau de sa partie métaphysaire, une tête généralement sphérique, apte à coopérer en appui, soit directement avec la glène, lorsqu'elle est en bon état, soit avec un implant glénoïdien, dans le cas contraire.

Différentes solutions ont été proposées pour tenter de se rapprocher le plus possible de l'anatomie naturelle de l'articulation glénohumérale de l'épaule. Notamment, il s'avère important que l'articulation de l'épaule ne soit pas contrainte, c'est-à-dire que la tête humérale ne soit pas contrainte dans la glène, mais roule et glisse sur cette dernière. Un bon fonctionnement de l'épaule est basé sur l'équilibre des forces générées par l'ensemble musculaire.

Des études anatomiques ont montré d'importantes variations d'un sujet à l'autre. L'anatomie d'un humérus peut essentiellement se définir par l'inclinaison humérale qui est de l'ordre de 130 à 140° et par la rétrotorsion humérale qui est l'angle que fait l'axe médian de la tête humérale avec l'axe de condyle du coude. Cette angle de rétrotorsion humérale est variable avec une amplitude qui peut aller de 5 à 45°, alors que la glène peut passer de 0 à 15° de rétroversion.

Il s'avère particulièrement important, pour assurer la stabilité de l'élément prothétique, de tenir compte de l'orientation normale des surfaces articulaires, qui est la condition indispensable à un travail satisfaisant et stable de la musculature de la coiffe.

Par le brevet EP 0549480, on connaît une prothèse humérale modulaire composée de trois éléments indépendants, à savoir une tige humérale, une entretoise intermédiaire et une calotte sphérique humérale. L'entretoise intermédiaire est conformée pour être ajustée sur la face d'appui de la tige humérale. Cette entretoise présente une portée tronconique apte à recevoir, avec capacité de réglage angulaire, la calotte sphérique qui est montée d'une manière excentrée. L'élément intermédiaire, constitué par l'entretoise, n'est pas monté avec capacité de déplacement par rapport à la tige humérale, pour modifier à volonté, la rétrotorsion humérale.

Les différents types de prothèses proposés à ce jour, ne permettent donc pas de modifier si nécessaire, l'angle de rétrotorsion, pour jouer sur la tension musculaire.

L'invention s'est fixée pour but de remédier à ces inconvénients, de manière simple, sûre, efficace et rationnelle.

Le problème que se propose de résoudre l'invention est de pouvoir contrôler la rétrotorsion et la tension musculaire, en considérant les paramètres suivants :
- positionnement de la prothèse en rétroversion correcte,
- reconstitution de la longueur normale de l'humérus,
- rayon de courbure et épaisseur de la tête,
- rapports entre têtes et trochanters,
- rayon de courbure longitudinal et transversal de la glène.

Pour résoudre un tel problème, et modifier à volonté l'angle de rétrotorsion, il a été conçu et mis au point un ensemble prothétique modulaire pour l'articulation de l'épaule, qui comprend, de manière indépendante, une gamme de tiges humérales, une gamme d'éléments intermédiaires et interchangeables et une gamme de têtes humérales, chaque élément étant constitué par un indexeur qui comprend un corps apte à être positionné, avec capacité de déplacement circulaire horizontal, dans un logement de forme complémentaire formé dans la partie métaphysaire de chaque tige humérale, ledit corps recevant, d'une manière excentrée, un col, disposé angulairement pour correspondre très sensiblement à l'inclinaison humérale anatomique, pour recevoir la tête humérale, l'orientation angulaire de l'ensemble de l'indexeur permettant de modifier, à volonté, la rétrotorsion humérale.

Pour résoudre le problème posé d'assurer la liaison entre l'indexeur et la tige humérale, le corps de l'indexeur a une forme tronconique, pour assurer un auto-blocage dans le logement correspondant, formé dans la partie métaphysaire de la tige.

Pour augmenter la sécurité de la fixation, le corps de l'indexeur reçoit une vis de fixation coopérant avec le logement.

Pour résoudre le problème de modifier la rétrotorsion, la face de dessus du corps, recevant le col de fixation de la tête, est inclinée d'une manière correspondante à l'inclinaison de la partie proximale de la tige et présente des moyens de repèrage aptes à être mis en correspondance avec un moyen d'indexation que présente une colerette d'appui.

Pour résoudre le problème posé d'assurer la fixation de la tête humérale par rapport à la tige, en combinaison avec l'organe indexeur, chaque tête humérale présente un logement apte à coopérer avec le col de l'indexeur et un ergot coopérant avec une partie de l'indexeur pour le blocage angulaire de ladite tête.

Pour résoudre le problème posé de restituer les fonctions de glissement de la tête humérale naturelle,

la gamme de têtes humérales comprend, d'une part, des têtes hémisphériques pour une coiffe des rotateurs parfaitement saine et, d'autre part, des têtes à double courbure pour une coiffe des muscles rotateurs hypovalides.

La double courbure est déterminée pour créer une surface de contacts importante entre la tête et l'élément glénoïdien aux environs de 80° d'abduction.

En ce qui concerne l'élément glénoïdien, ce dernier est soit constitué par un corps monobloc en polyéthylène, soit constitué par une embase métallique recevant un plateau d'appui en polyéthylène.

L'invention est exposée, ci-après plus en détail à l'aide des dessins annexés, dans lesquels :

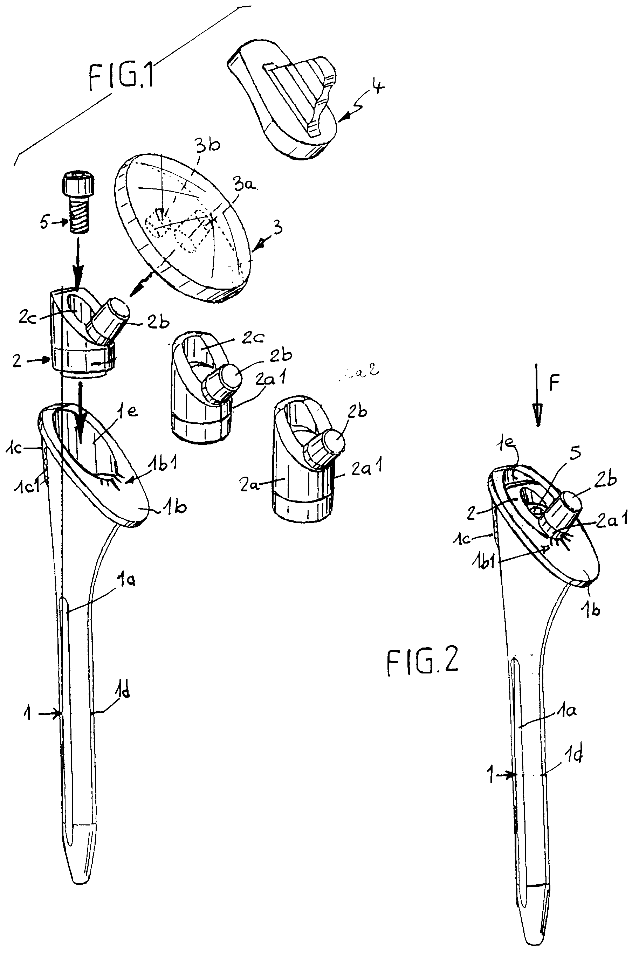

La figure 1 est une vue en perspective avant montage des principaux éléments constituant l'ensemble prothétique selon l'invention.
La figure 2 est une vue en perspective après assemblage des éléments.
La figure 3 est une vue en coupe montrant l'impaction de l'élément prothétique.
Les figures 4 et 5 sont des vues en plan montrant le réglage de la rétrotorsion.

Selon l'invention, l'ensemble prothétique pour l'articulation de l'épaule comprend :
- une gamme de tiges humérales (1),
- une gamme d'organes indexeurs (2),
- une gamme de têtes (3),
- une gamme d'éléments glénoïdiens (4).

La tige (1) est réalisée en plusieurs diamètres et en différentes longueurs, en considérant la moyenne pondérée d'une étude effectuée sur les coupes transversales de la métaphyse de plusieurs humérus. Cette tige présente un corps (1a) apte à être impacté dans le canal médulaire de l'humérus. La partie proximale de cette tige présente, au niveau de sa métaphyse, une colerette d'appui inclinée (1b), apte à coopérer avec la partie réséquée de l'humérus.

Cette tige peut présenter une ailette de réinsertion (1c) avec une série de trous (1c1) pour le positionnement et le maintien des fragments osseux. Cette ailette postérieure permet également d'assurer l'orientation lors du positionnement de la tige (1), en augmentant la stabilité rotationnelle.

En outre, cette tige présente sur une partie de sa hauteur, des nervures longitudinales (1d) permettant de mieux compacter le ciment et d'en laisser s'échapper les quantités en excès. Cette tige cannelée augmente la stabilité à la torsion du canal médulaire.

Chaque indexeur (2) comprend un corps (2a) apte à être positionné avec capacité de déplacement circulaire horizontal, dans un logement de forme complémentaire (1e), formé dans la partie métaphysaire de la tige humérale (1). Le corps (2a) présente un col latéralisé (2b), disposé angulairement pour correspondre très sensiblement à l'inclinaison humérale et anatomique. Ce col (2b) est destiné à recevoir la tête humérale (3), comme il sera indiqué dans la suite de la description.

Le corps (2a) a une forme tronconique pour assurer un auto-blocage dans le logement correspondant (1e) de la tige humérale. Le corps (2a) présente un moyen d'indication visuel (2a1) apte à être mis en correspondance avec des repères (1b1) que présente la colerette d'appui (1b), pour indiquer en conséquence le positionnement angulaire du col (2b). Le corps (2a) est réalisé selon différentes hauteurs.

Pour améliorer la fixation et éviter tout problème, l'organe indexeur (2) est maintenu dans le logement (1e) formé dans la partie métaphysaire de la tige (1), au moyen d'une vis (5). Cette vis (5) est engagée dans un trou (2c) pour traverser le corps (2a) et être vissée dans le fond du logement (1e). A noter que la face de dessus (2a2) du corps (2a) est inclinée d'une manière correspondante à l'inclinaison de la colerette (1b) de la tige.

Le col (2b), de forme tronconique, coopère avec un logement de forme complémentaire (3a) formé dans l'épaisseur de la tête humérale (3). Le blocage en rotation de la tête (3) sur l'organe indexeur (2) s'effectue au moyen d'un ergot (3b) coopérant avec une partie de l'indexeur (2), notamment le trou (2c).

Selon l'invention, la tête humérale est réalisée en plusieurs épaisseurs et selon deux modes d'exécution principaux.

Selon un premier mode, la tête (3) est sphérique, dans le cas d'une coiffe parfaitement saine.

Selon une second mode, dans le cas d'une coiffe des muscles rotateurs hypovalides, la tête sphérique (3) présente une double courbure. Cette double courbure est déterminée pour créer une surface de contact importante, entre la tête et la glène, qui se réalise aux environs de 80° d'abduction. Cette double courbure résulte d'une simulation sur ordinateur.

A noter que l'épaisseur et le rayon de courbure de la tête humérale ne doivent pas être inférieurs à ceux de l'épiphyse du patient.

A titre indicatif, la tête humérale hémisphérique est disponible en trois dimensions de diamètre différent et selon trois épaisseurs différentes. L'élément huméral à double courbure peut varier également en diamètre et en épaisseur. Le fait que la tête humérale (3) soit séparée de la tige (1), offre la possibilité de la remplacer au cours de l'opération par des têtes de dimensions différentes et correspondant mieux aux exigences fonctionnelles constatées. Ces dispositions permettent également une reprise chirurgicale plus facile en cas de substitution de la glène.

En ce qui concerne l'élément glénoïdien (4), ce dernier peut être réalisé en polyéthylène haute densité et fixé au moyen de ciment, en étant disponible en deux épaisseurs. On prévoit également de réaliser l'élément glénoïdien de manière connue, avec une embase en titane, apte à recevoir un plateau d'appui en polyéthylène. L'embase est fixée dans la cavité, au moyen par exemple, d'une vis en combinaison avec des pivots périphériques.

L'utilisation de l'ensemble prothétique selon l'invention s'avère particulièrement important et avantageux.

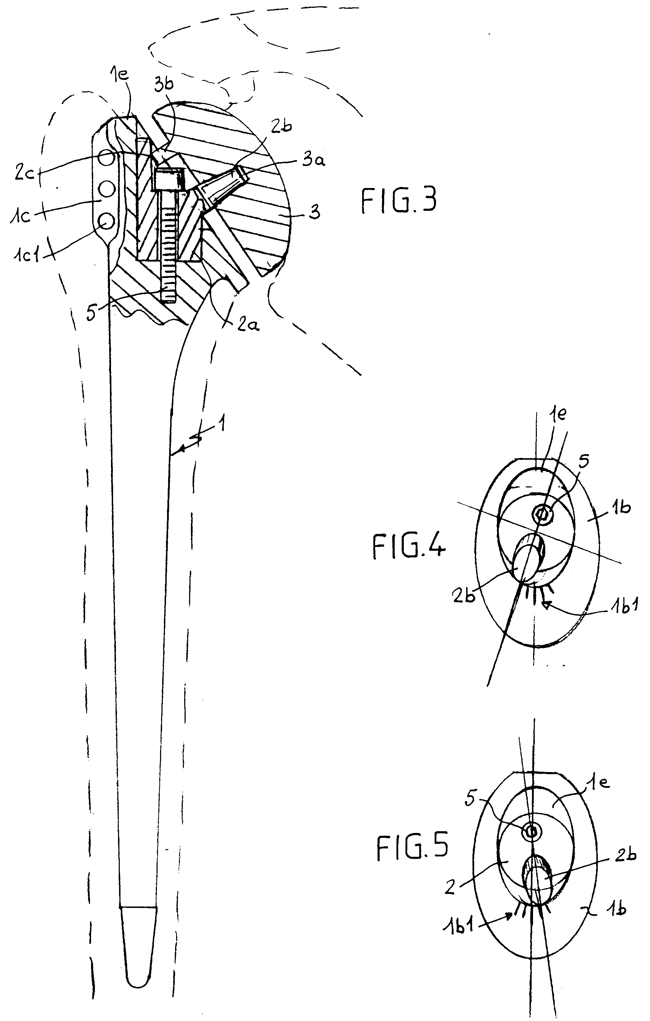

L'opérateur met en place dans le canal médulaire, la tige humérale (1) en l'équipant de l'indexeur (2), qui est orienté en position intermédiaire zéro. Après avoir positionné la tige équipé de l'indexeur (2), des têtes humérales d'essais (3) sont mises en place sur le col (2b) de l'indexeur, afin de contrôler la rétrotorsion et la tension musculaire. Si la rétrotorsion n'est pas satisfaisante, le chirurgien démonte la tête (3) et modifie la rétrotorsion en tournant l'indexeur de quelques degrés en se repérant par rapport au repère (1b1) de la colerette (1b) de la tige (1) (figures 4 et 5). Lorsque le bon réglage est trouvé, l'organe indexeur est bloqué en position au moyen de la vis (5).

La tension musculaire est réglée en choisissant la bonne épaisseur de la tête humérale (3), qui est impactée sur le col (2b), en étant bloqué angulairement par son ergot (3b).

Les avantages ressortent bien de la description.

## Revendications

1. Ensemble prothétique modulaire pour l'articulation de l'épaule, comprenant, de manière indépendante, une gamme de tiges humérales (1), une gamme d'éléments intermédiaires et interchangeables (2) et une gamme de têtes humérales (3), caractérisé en ce que chaque élément (2) est constitué par un indexeur qui comprend un corps (2a) apte à être positionné, avec capacité de déplacement circulaire horizontal, dans un logement (1e) de forme complémentaire formé dans la partie métaphysaire de chaque tige humérale (1), ledit corps (2a) recevant, d'une manière excentrée, un col (2b), disposé angulairement pour correspondre très sensiblement à l'inclinaison humérale anatomique, pour recevoir la tête humérale (3), l'orientation angulaire de l'ensemble de l'indexeur permettant de modifier, à volonté, la rétrotorsion humérale.

2. Ensemble selon la revendication 1, caractérisé en ce que le corps (2a) de l'indexeur (2) a une forme tronconique, pour assurer un auto-blocage dans le logement correspondant (1e), formé dans la partie métaphysaire de la tige.

3. Ensemble selon la revendication 1, caractérisé en ce que le corps (2a) de l'indexeur reçoit une vis de fixation (5) coopérant avec le logement.

4. Ensemble selon la revendication 1, caractérisé en ce que la face de dessus (2a2) du corps (2a), recevant le col de fixation (2b) de la tête, est inclinée d'une manière correspondante à l'inclinaison de la partie proximale de la tige.

5. Ensemble selon la revendication 1, caractérisé en ce que chaque tête humérale présente un logement (3a) apte à coopérer avec le col (2b) de l'indexeur (2) et un ergot (3b) coopérant avec une partie de l'indexeur pour le blocage angulaire de ladite tête et la face de dessous du corps présente des moyens de repèrage aptes à être mis en correspondance avec un moyen d'indexation que présente une colerette d'appui (1b).

6. Ensemble selon la revendication 1, caractérisé en ce que la gamme de têtes humérales comprend, d'une part, des têtes hémisphériques pour une coiffe des rotateurs parfaitement saine et, d'autre part, des têtes à double courbure pour une coiffe des muscles rotateurs hypovalides.

7. Ensemble selon la revendication 6, caractérisé en ce que la double courbure est déterminée pour créer une surface de contacts importante entre la tête et un élément glénoïdien (4) aux environs de 80° d'abduction.

8. Ensemble selon la revendication 1, caractérisé en ce qu'un élément glénoïdien (4) est constitué par un corps monobloc en polyéthylène.

9. Ensemble selon la revendication 1, caractérisé en ce qu'un élément glénoïdien (4) est constitué par une embase métallique recevant un plateau d'appui en polyéthylène.

## Claims

1. A modular prosthetic assembly for the shoulder joint, comprising, in independent manner, a range of humerus rods (1), a range of interchangeable intermediate elements (2), and a range of humerus heads (3), the assembly being characterized in that each element (2) is constituted by an indexer comprising a body (2a) suitable for being positioned with the ability for horizontal circular displacement, in a housing (1e) of complementary shape formed in the metaphyseal portion of each humerus rod (1), said body (2a) receiving, eccentrically, a neck (2b) disposed angularly to correspond very closely to the anatomical inclination of the humerus to receive the humerus head (3), the angular orientation of the indexer assembly making it possible, at will, to modify the retrotorsion of the humerus.

2. An assembly according to claim 1, characterized in that the body (2a) of the indexer (2) is frustoconical in shape, to provide self-locking in the corresponding housing (1e) formed in the metaphyseal portion of the rod.

3. An assembly according to claim 1, characterized in that the body (2a) of the indexer receives a fixing screw (5) which co-operates with the housing.

4. An assembly according to claim 1, characterized in that the top face (2a, 2) of the body (2a) receiving the fixing neck (2b) for the head, is inclined so as to correspond to the inclination of the proximal portion of the rod.

5. An assembly according to claim 1, characterized in that each humerus head has a housing (3a) suitable for co-operating with the neck (2b) of the indexer (2) and a stud (3b) co-operating with a portion of the indexer to lock said head angularly, and the bottom face of the body has position-identifying means suitable for being put into correspondence with indexing means on a thrust collar (1b).

6. An assembly according to claim 1, characterized in that the range of humerus heads comprises firstly hemispherical heads to be covered by rotators that are perfectly healthy, and secondly heads of double curvature to be covered by rotator muscles that are hypovalid.

7. An assembly according to claim 6, characterized in that the double curvature is designed to create a large contact area between the head and a glenoid element (4) at about 80° abduction.

8. An assembly according to claim 1, characterized in that a glenoid element (4) is constituted by a one-piece body of polyethylene.

9. An assembly according to claim 1, characterized in that a glenoid element (4) is constituted by a metal base receiving a polyethylene thrust plate.

## Patentansprüche

1. Modularer Prothesensatz für das Schultergelenk, der unabhängig ein Sortiment von Oberarmstangen (1), ein Sortiment von zwischengeordneten und austauschbaren Elementen (2) und ein Sortiment von Oberarmköpfen (3) aufweist, dadurch gekennzeichnet, daß jedes Element (2) von einem Positionierungsglied gebildet ist, das einen Körper (2a) aufweist, der dazu eingerichtet ist, mit der Fähigkeit zur kreisförmigen, horizontalen Verlagerung in einem Sitz (1e) mit komplementärer Form angeordnet zu werden, der im Metaphyse-Abschnitt einer jeden Oberarmstange (1) ausgebildet ist, wobei der genannte Körper (2a) exzentrisch einen Hals (2b) aufnimt, der winklig angeordnet ist, um sehr deutlich der anatomischen Oberarmneigung zu entsprechen, um den Oberarmkopf (3) aufzunehmen, wobei die winklige Ausrichtung der Positionierungsglied-Baugruppe es gestattet, wuschgemäß die Oberarm-Rückwärtsdrehung zu modifizieren.

2. Satz nach Anspruch 1, dadurch gekennzeichnet, daß der Körper (2a) des Positioniformerungsgliedes (2) Kegelstumpfform hat, um eine Selbstsperrung im entsprechenden Sitz (1e) sicherzustellen, der im Metaphys-Abschnitt der Stange ausgebildet ist.

3. Satz nach Anspruch 1, dadurch gekennzeichnet, daß der Körper (2a) des Positionierungsgliedes eine Befestigungsschraube (15) aufnimmt, die mit dem Sitz zusammenwirkt.

4. Satz nach Anspruch 1, dadurch gekennzeichnet, daß die obere Fläche (2a2) des Körpers (2a), der den Befestigungshals (2b) aufnimmt, entsprechend der Neigung des proximalen Abschnitts der Stange geneigt ist.

5. Satz nach Anspruch 1, dadurch gekennzeichnet, daß jeder Oberarmkopf einen Sitz (3a) aufweist, der dazu eingerichtet ist, mit dem Hals (2b) des Positionierungsgliedes (2) zusammenzuwirken, sowie eine Nase (3b), die mit einem Abschnitt des Positionierungsgliedes für die Winkelblockierung des genannten Kopfes zusammenwirkt, und daß die obere Fläche am Körper Mittel zur Markierung aufweist, die dazu eingerichtet sind, mit Positionierungmitteln in Übereinstimmung gebracht zu werden, die ein Anflageflansch (1b) darbietet.

6. Satz nach Anspruch 1, dadurch gekennzeichnet, daß das Sortiment von Oberarmköpfen einerseits halbkugelige Köpfe für eine vollkommen gesunde Rotatorenmanschette und andererseits Köpfe mit doppelter Krümmung für eine Manschette für entkräftete Rotatorenmuskeln aufweist.

7. Satz nach Anspruch 6, dadurch gekennzeichnet, daß die doppelte Krümmung dazu bestimmt ist, eine wesentliche Berührungsfläche zwischen dem Kopf und einem Gelenkpfannenelement (4) mit etwa 80° Abduktion zu erzeugen.

8. Satz nach Anspruch 1, dadurch gekennzeichnet, daß ein Gelenkpfannenelement (4) von einem einstückigen Polyethylenkörper gebildet ist.

9. Satz nach Anspuch 1, dadurch gekennzeichnet, daß ein Gelenkpfannenelement (4) von einem Metallsockel gebildet ist, der eine Auflageplatte aus Polyethylen aufnimmt.
